# EUROPEAN PATENT APPLICATION

(11) **EP 4 332 083 A1**
(43) Date of publication of application: **06.03.2024**
(21) Application number: 23150544.7
(22) Date of filing: 06.01.2023
(51) Int. Cl.: C07C 63/04, G03F 7/004

(54) **CARBOXYLATE SALT, PHOTORESIST COMPOSITION INCLUDING THE SAME, AND METHOD OF FORMING PATTERN BY USING THE PHOTORESIST COMPOSITION**

(30) Priority: 29.08.2022 KR 20220108657
(71) Applicant: Samsung Electronics Co., Ltd., Gyeonggi-do 16677 (KR)
(72) Inventor: CHAE, Jungha, 16678 Suwon-si (KR); KOH, Haengdeog, 16678 Suwon-si (KR); KWAK, Yoonhyun, 16678 Suwon-si (KR); KIM, Minsang, 16678 Suwon-si (KR); KIM, Hana, 16678 Suwon-si (KR); KIM, Hyeran, 16678 Suwon-si (KR); NAM, Youngmin, 16678 Suwon-si (KR); LEE, Changheon, 16678 Suwon-si (KR); IM, Kyuhyun, 16678 Suwon-si (KR)
(74) Representative: Elkington and Fife LLP

(57) **Abstract**

Provided are a carboxylate salt represented by Formula 1, a photoresist composition including the carboxylate salt, and a method of forming a pattern by using the photoresist composition 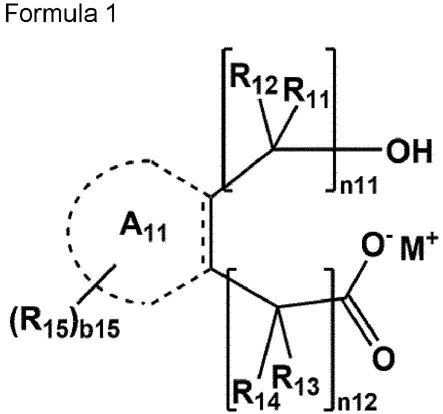 wherein, in Formula 1, A₁₁, R₁₁ to R₁₅, b15, n11, n12, and M⁺ are described in the specification.

## Description

### FIELD OF THE INVENTION

The disclosure relates to a carboxylate salt, a photoresist composition including the same, and a method of forming a pattern by using the photoresist carboxylic acid.

### BACKGROUND OF THE INVENTION

In manufacturing a semiconductor, photoresists of which physical properties change in response to light are used to form fine patterns. Among photoresists, chemically amplified photoresists have been widely used. A chemically amplified photoresist enables patterning by changing the solubility of a base resin in a developing solution by reacting to an acid, which is formed by a reaction between light and a photoacid generator, with the base resin.

However, in the case of a chemically amplified photoresist, problems such as a decrease in pattern uniformity as the formed acid diffuses to an unexposed area and/or an increase in surface roughness may be caused. A quencher may be used to solve or mitigate such problems, but the use of a quencher may cause a problem of increasing a dose requirement for the light exposure.

Accordingly, there is a need for a quencher that can be effective, even with a small amount, and has improved dispersibility and/or improved compatibility with the base resin.

### SUMMARY OF THE INVENTION

Provided are a carboxylate salt capable of serving as a quencher having improved dispersibility and/or improved compatibility, a photoresist composition including the carboxylate salt, and a method of forming a pattern by using the photoresist composition.

Additional aspects will be set forth in part in the description which follows and, in part, will be apparent from the description, or may be learned by practice of the presented embodiments of the disclosure.

According to an aspect of the disclosure, a carboxylate salt represented by Formula 1 may be provided:

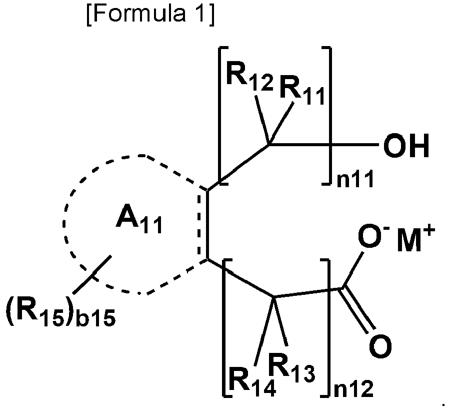

In Formula 1, A₁₁ may be a cyclic C₁-C₂₀ hydrocarbon group that may optionally include a heteroatom, and 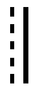 represents at least one of a carbon-carbon single bond or a carbon-carbon double bond, R₁ to R₁₅ may each independently be at least one of hydrogen, deuterium, a halogen, or a linear, branched, or cyclic C₁-C₂₀ monovalent hydrocarbon group that may optionally include a heteroatom, b15 may be an integer from 0 to 10, n11 and n12 may each be 0, 1, or 2 such that the sum of n11 and n12 may be 1 or 2, and M⁺ may be a substituted or unsubstituted sulfonium cation, a substituted or unsubstituted iodonium cation, or a substituted or unsubstituted ammonium cation.

According to another aspect of the disclosure, a photoresist composition includes the carboxylate salt, an organic solvent, a base resin, and a photoacid generator.

According to another aspect of the disclosure, a method of forming a pattern includes forming a photoresist film by applying the photoresist composition, exposing at least a portion of the photoresist film with high energy radiation, and developing the exposed photoresist film by using a developer solution.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a flowchart illustrating a method of forming a pattern according to an embodiment; and
FIG. 2 is a side cross-sectional view illustrating a method of forming a pattern according to an embodiment.

### DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figure, to explain aspects. As used herein, the term "and/or" includes any and all combinations of one or more of the associated listed items. Expressions such as "at least one of," when preceding a list of elements, modify the entire list of elements and do not modify the individual elements of the list.

Terms such as "first", "second", "third", and/or the like may be used to describe various components but are used only for the purpose of distinguishing one component from other components, and the order, type, and/or the like of the components are not limited.

It will be understood that when a component, such as a layer, a film, a region, or a plate, is referred to as being "on" or "above" another component in the specification, the component can directly contact to be above, below, right, or left of the another component as well as being above, below, left, or right of the another component in a non-contact manner.

An expression used in the singular encompasses the expression of the plural, unless it has a clearly different meaning in the context. It is to be understood that the terms such as "including," "having," and "comprising" are intended to indicate the existence of the features, numbers, steps, actions, components, parts, ingredients, materials, or combinations thereof disclosed in the specification, and are not intended to preclude the possibility that one or more other features, numbers, steps, actions, components, parts, ingredients, materials, or combinations thereof may exist or may be added.

Whenever a range of values is enumerated, the range includes all values within the range as if recorded explicitly clearly and may further include the boundaries of the range. Accordingly, the expression in a range of "X" to "Y" includes all values between X and Y, including X and Y. When the terms "about" or "substantially" are used in this specification in connection with a numerical value, it is intended that the associated numerical value includes a manufacturing tolerance (e.g., ±10%) around the stated numerical value. Further, regardless of whether numerical values are modified as "about" or "substantially," it will be understood that these values should be construed as including a manufacturing or operational tolerance (e.g., ±10%) around the stated numerical values.

Hereinafter, the disclosure will be described in detail by explaining embodiments with reference to the accompanying drawings, and substantially identical or corresponding components are given the same reference numerals in the drawings, and thus a description thereof will be omitted. In the drawings, thicknesses are enlarged to clearly represent various layers and regions. In the drawings, thicknesses of some layers and regions are exaggerated for convenience of description. Meanwhile, embodiments described below are illustrative examples of embodiments, and various changes in forms and details may be made.

### [Carboxylate salt]

A carboxylate salt according to one or more embodiments may be represented by Formula 1:

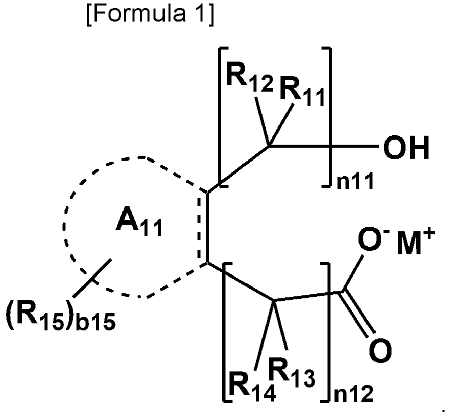

In Formula 1, A₁₁ may be a cyclic C₁-C₂₀ hydrocarbon group that optionally includes a heteroatom, and 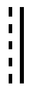 represents a carbon-carbon single bond and/or a carbon-carbon double bond, R₁₁ to R₁₅ may each independently be at least one of hydrogen; deuterium; a halogen; or a linear, branched, or cyclic C₁-C₂₀ monovalent hydrocarbon group that optionally includes a heteroatom,b15 may be an integer from 0 to 10, n11 and n12 may each be 0, 1, or 2, and the sum of n11 and n12 may be 1 or 2, and M+ may be a substituted or unsubstituted onium cation (such as at least one of a substituted or unsubstituted sulfonium cation, a substituted or unsubstituted iodonium cation, a substituted or unsubstituted ammonium cation, and/or the like). In one or more embodiments, the linear, branched, or cyclic C₁-C₂₀ monovalent hydrocarbon group may optionally include a heteroatom.

In the description of A₁₁ in Formula 1, the term "cyclic C₁-C₂₀ hydrocarbon group" as used herein may include, for example, at least one of a saturated cycloaliphatic hydrocarbon group (e.g., a cyclopentane group, a cyclohexane group, a cyclopentylmethane group, a cyclopentylethane group, a cyclopentylbutane group, a cyclohexylmethane group, a cyclohexylethane group, a cyclohexylbutane group, an adamantane group, a 1-adamantylmethane group, a norbornane group, a norbornylmethane group, a tricyclodecane group, a tetracyclododecane group, a tetracyclododecanylmethane group, a dicyclohexylmethane group, etc.); an unsaturated cycloaliphatic hydrocarbon group (e.g., a cyclopentene group, a cyclopentadiene group, a cyclohexene group, a cyclohexadiene group, etc.); an arene group (e.g., a benzene group, a naphthalene group, and/or the like); a heteroatom-containing hydrocarbon group (e.g., a tetrahydrofuran group, a 4-oxo-1-adamantane group, a 3-oxocyclohexane group, etc.), and/or the like. In addition, among these groups, some hydrogen atoms may be substituted with a moiety including a heteroatom (such as oxygen, sulfur, nitrogen, a halogen atom, etc.) and/or some carbon atoms may be replaced by a moiety including a heteroatom (such as oxygen, sulfur, nitrogen, etc.). Accordingly, these groups may each include a hydroxy group, a cyano group, a carbonyl group, a carboxyl group, an ether linkage, an ester linkage, a sulfonic ester linkage, a carbonate, a lactone ring, a sultone ring, a carboxylic anhydride moiety, a haloalkyl moiety, and/or the like.

For example, in Formula 1, A₁₁ may be a C₁-C₁₀ cyclic hydrocarbon group. That is in one or more embodiments, in Formula 1, A₁₁ may not include a heteroatom.

In one or more embodiments, in Formula 1, A₁₁ may be at least one of a cyclopentane group, a cyclopentene group, a cyclopentadiene group, a cyclohexane group, a cyclohexene group, a cyclohexadiene group, a benzene group, a naphthalene group, and/or the like.

In one or more embodiments, in Formula 1, A₁₁ may be a at least one of a cyclopentane group, a cyclohexane group, a benzene group, a naphthalene group, and/or the like.

Regarding the description of R₁₁ to R₁₅ in Formula 1, the monovalent hydrocarbon group may include, for example, at least one of a linear or branched alkyl group (e.g., a methyl group, an ethyl group, a propyl group, an isopropyl group, a butyl group, an isobutyl group, a sec-butyl group, a tert-butyl group, a pentyl group, a neopentyl group, a hexyl group, a heptyl group, a 2-ethylhexyl group, a nonyl group, etc.); a monovalent saturated alicyclic hydrocarbon group (e.g., a cyclopentyl group, a cyclohexyl group, a cyclopentylmethyl group, a cyclopentylethyl group, a cyclopentylbutyl group, a cyclohexylmethyl group, a cyclohexylethyl group, a cyclohexylbutyl group, a 1-adamantyl group, a 2-adamantyl group, a 1-adamantylmethyl group, a norbornyl group, a norbornylmethyl group, a tricyclodecanyl group, a tetracyclododecanyl group, a tetracyclododecanylmethyl group, a dicyclohexylmethyl group, etc.); a monovalent unsaturated aliphatic hydrocarbon group (e,g., an allyl group, a 3-cyclohexenyl group, etc.); an aryl group (e.g., a phenyl group, a 1-naphthyl group, a 2-naphthyl group, etc.); an arylalkyl group (e.g., a benzyl group, a diphenylmethyl group, etc.); a heteroatom-containing monovalent hydrocarbon group (e.g., a tetrahydrofuranyl group, a methoxymethyl group, an ethoxymethyl group, a methylthiomethyl group, an acetamidemethyl group, a trifluoroethyl group, a (2-methoxyethoxy)methyl group, an acetoxymethyl group, a 2-carboxy-1-cyclohexyl group, a 2-oxopropyl group, a 4-oxo-1-adamantyl group, a 3-oxocyclohexyl group, etc.), and/or the like. In addition, among these groups, some hydrogen atoms may be substituted with a moiety including a heteroatom (such as oxygen, sulfur, nitrogen, a halogen atom, etc.), and/or some carbon atoms may be replaced by a moiety including a heteroatom (such as oxygen, sulfur, nitrogen, etc.). Accordingly, these groups may each include a hydroxy group, a cyano group, a carbonyl group, a carboxyl group, an ether linkage, an ester linkage, a sulfonic ester linkage, a carbonate, a lactone ring, a sultone ring, a carboxylic anhydride moiety, a haloalkyl moiety, and/or the like.

For example, in one or more embodiments, in Formula 1, R₁₁ to R₁₅ may each independently be hydrogen; deuterium; fluorine (F); and/or a methyl group, an ethyl group, a propyl group, an isopropyl group, a butyl group, an isobutyl group, a sec-butyl group, a tert-butyl group, a pentyl group, a neopentyl group, a hexyl group, a heptyl group, a 2-ethylhexyl group, an n-nonyl group, a cyclopentyl group, a cyclohexyl group, a cyclopentylmethyl group, a cyclopentylethyl group, a cyclopentylbutyl group, a cyclohexylmethyl group, a cyclohexylethyl group, a cyclohexylbutyl group, a 1-adamantyl group, a 2-adamantyl group, a 1-adamantylmethyl group, a norbornyl group, a norbornylmethyl group, a tricyclotricyclodecanyl group, a tetracyclododecanyl group, a tetracyclododecanylmethyl group, a dicyclohexylmethyl group, a phenyl group, etc., each unsubstituted or substituted with F.

In one or more embodiments, in Formula 1, R₁₁ to R₁₅ may each independently be hydrogen, deuterium, F, CH₂F, CHF₂, or CF₃.

In one or more embodiments, in Formula 1, n11 and n12 may each be 0 or 1, wherein the sum of n11 and n12 may be 1.

In one or more embodiments, the carboxylate salt represented by Formula 1 may be represented by at least one of Formulae 1-1 to 1-5:

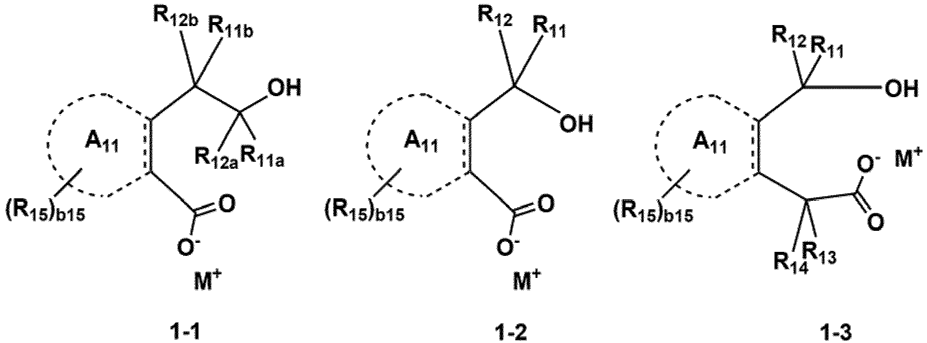

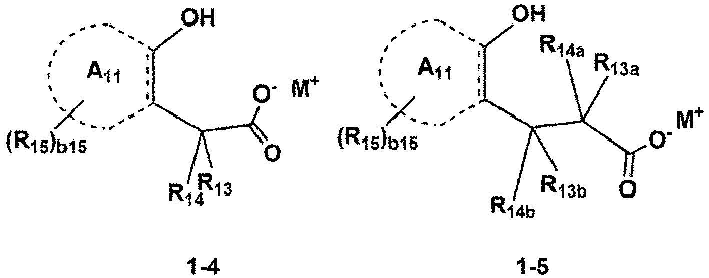

In Formulae 1-1 to 1-5, A₁₁, R₁₁ to R₁₅, b15, and M+ may each be the same as defined in Formula 1, and R₁₁ₐ, R₁₂ₐ, R₁₃ₐ, R₁₄ₐ, R_{11b}, R_{12b}, R_{13b}, and R_{14b} may each be the same as defined in connection with R₁₁ in Formula 1.

In one or more embodiments, in Formulae 1-1 to 1-5, A₁₁ may be a C₁-C₁₀ cyclic hydrocarbon group.

For example, in one or more embodiments, in Formulae 1-1 to 1-5, A₁₁ may be a cyclopentane group, a cyclopentene group, a cyclopentadiene group, a cyclohexane group, a cyclohexene group, a cyclohexadiene group, a benzene group, a naphthalene group, etc.

In one or more embodiments, in Formulae 1-1 to 1-5, A₁₁ may be a cyclopentane group, a cyclohexane group, a benzene group, or a naphthalene group.

In one or more embodiments, the carboxylate salt represented by Formula 1 may be represented by at least one of Formulae 1-2 and 1-4.

In Formula 1, M+ may be represented by at least one of Formulae 3-1 to 3-3:

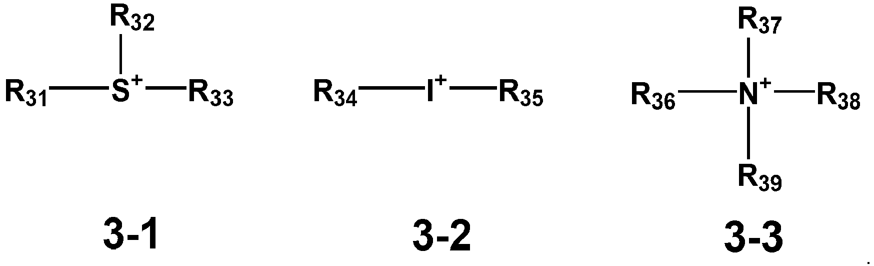

In Formulae 3-1 to 3-3, R₃₁ to R₃₉ may each independently be a linear, branched, or cyclic C₁-C₂₀ monovalent hydrocarbon group that optionally includes a heteroatom; two adjacent groups among R₃₁ to R₃₃ may optionally be bonded to each other to form a ring; R₃₄ and R₃₅ may optionally be bonded to each other to form a ring; and two adjacent groups among R₃₆ to R₃₉ may optionally be bonded to each other to form a ring.

The "monovalent hydrocarbon group" in Formulae 3-1 to 3-3 may be understood by referring to the "monovalent hydrocarbon group" among the examples of R₁₁ in Formula 1.

For example, in one or more embodiments, in Formulae 3-1 to 3-3, R₃₁ to R₃₅ may each independently be at least one of a C₆-C₂₀ aryl group unsubstituted or substituted with at least one of deuterium, a halogen, a hydroxyl group, a C₁-C₆ alkyl group, a C₁-C₆ halogenated alkyl group, a C₁-C₆ alkoxy group, a C₃-C₆ cycloalkyl group, and/or a C₃-C₆ cycloalkoxy group; R₃₆ to R₃₉ may each independently be a C₁-C₁₀ alkyl group unsubstituted or substituted with at least one of deuterium, a halogen, a hydroxyl group, a C₁-C₆ alkyl group, a C₁-C₆ alkoxy group, a C₃-C₆ cycloalkyl group, a C₃-C₆ cycloalkoxy group, and/or a C₆-C₁₀ aryl group; two adjacent groups among R₃₁ to R₃₃ may optionally be bonded to each other to form a ring; R₃₄ and R₃₅ may optionally be bonded to each other to form a ring; and/or two adjacent groups among R₃₆ to R₃₉ may optionally be bonded to each other to form a ring.

For example, in one or more embodiments, in Formula 1, M+ may be represented by at least one of Formulae 3-11 to 3-13:

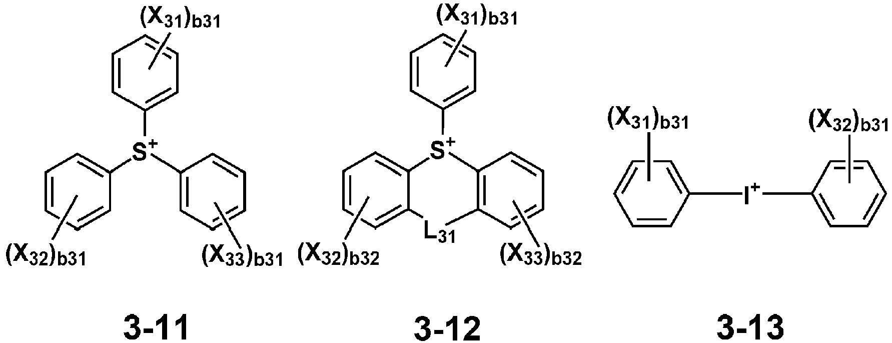

In Formulae 3-11 to 3-13, X₃₁ to X₃₃ may each independently be at least one of hydrogen, deuterium, a halogen, a C₁-C₆ alkyl group, and/or a C₁-C₆ halogenated alkyl group; b31 may be an integer from 1 to 5; b32 may be an integer from 1 to 4; L₃₁ may be a single bond, O, S, CO, SO, SO₂, CRR', or NR; and/or R and R' may each independently be hydrogen, deuterium, a halogen, a hydroxy group, a C₁-C₆ alkyl group, a C₁-C₆ halogenated alkyl group, a C₁-C₆ alkoxy group, a C₃-C₆ cycloalkyl group, and/or a C₃-C₆ cycloalkoxy group.

For example, in Formulae 3-11 to 3-13, X₃₁ to X₃₃ may each independently be hydrogen, F, I, or CF₃.

In one or more embodiments, the carboxylate salt represented by Formula 1 may be represented by one of Formulae 1-11 to 1-20:

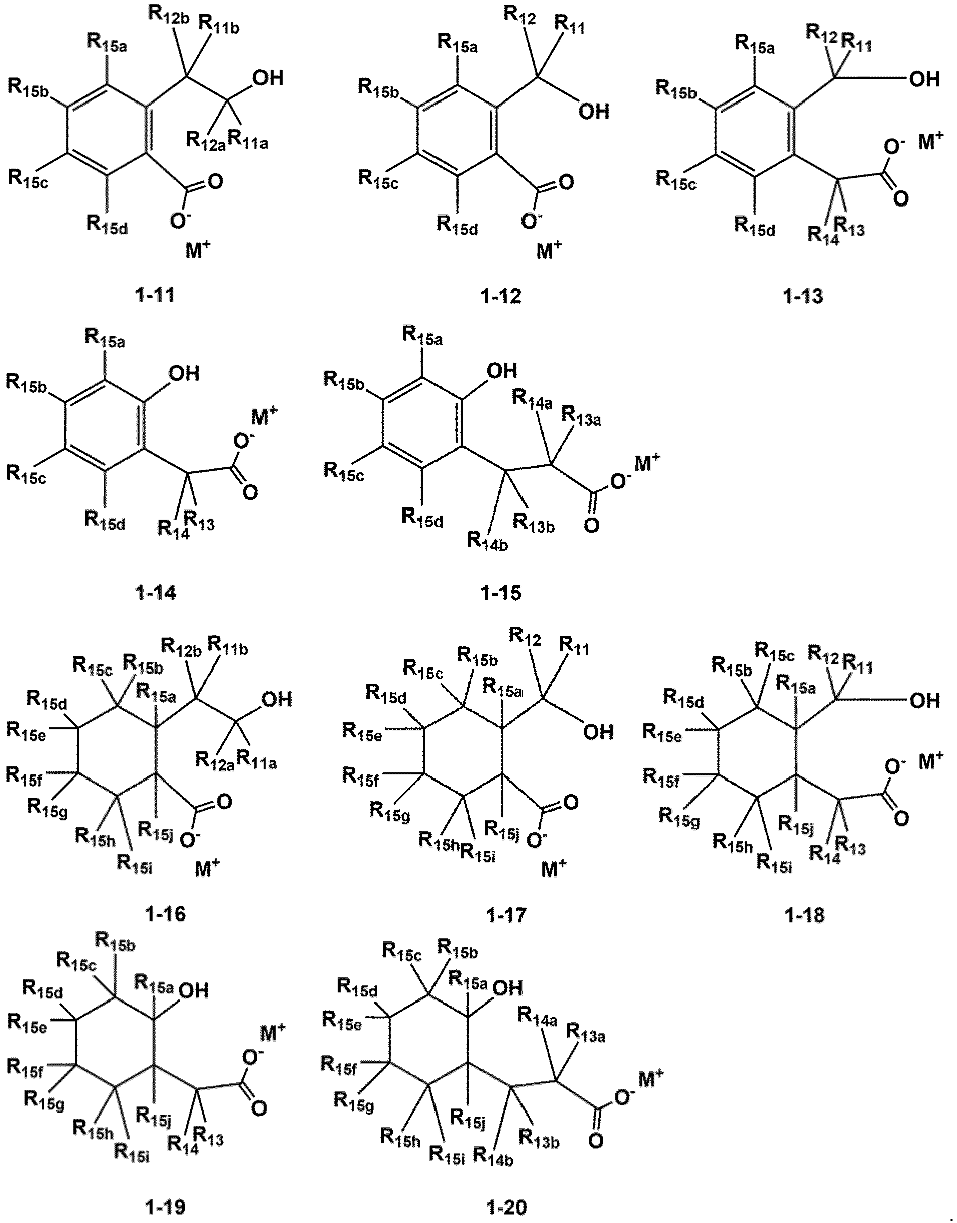

In Formulae 1-11 to 1-20, M⁺ may be the same as defined in Formula 1; and/or R₁₁ₐ, R₁₂ₐ, R₁₃ₐ, R₁₄ₐ, R_{11b}, R_{12b}, R_{13b}, R_{14b}, and R₁₅ₐ to R₁₅ⱼ may each be the same (or substantially similar) as defined in connection with R₁₁ in Formula 1.

In one or more embodiments, the carboxylate salt represented by Formula 1 may be represented by one of Formulae 1-12 and 1-14.

For example, in Formulae 1-11 to 1-20, M⁺ may be represented by one of Formulae 3-11 to 3-13.

In one or more embodiments, in Formulae 1-11 to 1-20, M+ may be represented by Formula 3-11.

In one or more embodiments, the carboxylate salt represented by Formula 1 may be selected from Group C:

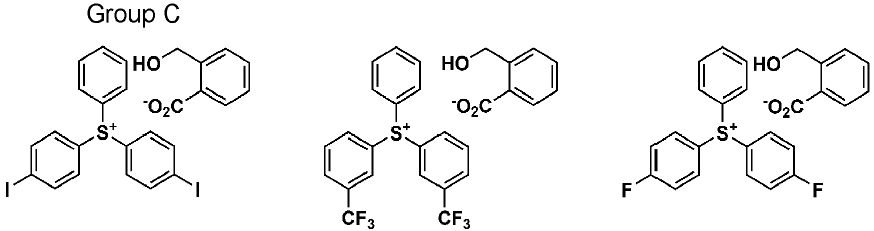

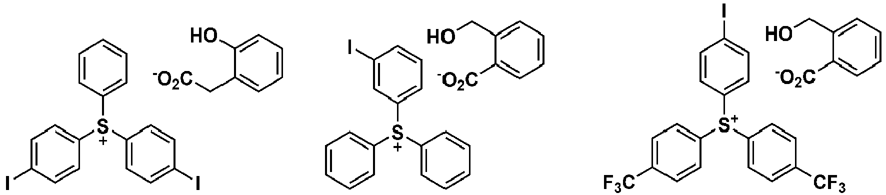

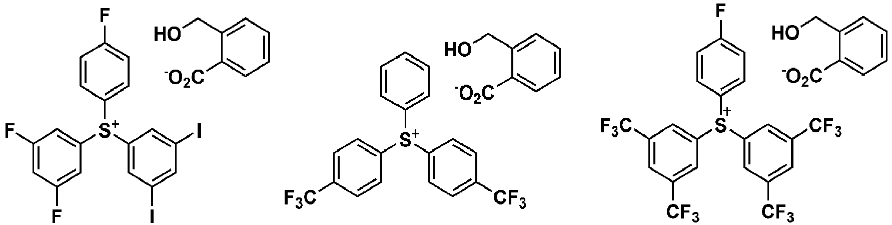

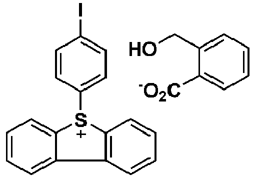

In general, when forming a pattern in a photoresist film using a photoresist composition, an acid (generated from a photoacid generator upon light exposure) may diffuse in the photoresist film. Accordingly, the acid may penetrate even to an unexposed area so that the sensitivity and/or resolution of the photoresist composition may be lowered. Therefore, to improve the sensitivity and/or resolution of the photoresist composition, a quencher may be used to effectively reduce the diffusion of the acid.

However, the use of a quencher may not simply reduce the diffusion of the acid. To enhance the effect of the quencher, while using the quencher in an appropriate amount, there is a need to improve the dispersion degree of the quencher and/or the compatibility of the quencher with a base resin.

For example, to improve the compatibility of the quencher with a base resin, a method of coupling macromolecules has been studied, but such a method could not solve problems of a decrease in the solubility of the quencher in an organic solvent and/or a decrease in the compatibility of the quencher with a base resin. In addition, to improve the compatibility of the quencher with a base resin, a method of binding the quencher to a base resin itself has been studied, but practical applications had many difficulties due to problems of a decrease in the solubility of the base resin itself and/or the influence of the quencher on the contrast.

In addition, when a low-molecular quencher is used, aggregation occurs in the base resin due to interactions among molecules of the low-molecular quencher (in particular, without being limited to a specific theory, interactions by electrostatic attraction among ion-binding molecules). Also, there is a problem that a small amount of the quencher is not typically enough to effectively reduce the diffusion of the acid.

However, when the carboxylate salt represented by Formula 1 is used as the quencher, due to increased attraction between heterogeneous molecules (compared to attraction between homogeneous molecules), the dispersibility of the quencher in the base resin may be improved, and thus the diffusion of the acid may be effectively reduced even with a small amount of the quencher.

In addition, generally due to a difference in the diffusion distance of the acid, typically the roughness of the surface of the photoresist film may increase after development. However, when a quencher having the carboxylate salt according to one or more embodiments is used, the diffusion of the acid may be effectively and evenly reduced, thereby improving the surface roughness.

### [Photoresist composition]

According to another aspect of the disclosure, a photoresist composition includes the carboxylate salt, an organic solvent, a base resin, and a photoacid generator. The photoresist composition may have properties including improved developability and/or improved resolution.

The solubility of the photoresist composition in a developing solution may be changed by exposure with high-energy rays. The photoresist composition may be a positive photoresist composition that forms a positive photoresist pattern after an exposed area of a photoresist film is dissolved and removed; or a negative photoresist composition that forms a negative photoresist pattern after an unexposed area of a photoresist film is dissolved and removed. In addition, a sensitive photoresist composition according to one or more embodiments may be for, e.g., an alkali development process using an alkali developing solution for the development treatment when forming a photoresist pattern formation or may be for a solvent development process using a developing solution containing an organic solvent (hereinafter also referred to as an organic developing solution) for the development treatment.

The carboxylate salt may be a photo-decomposable compound that is decomposable by light exposure. For example, the carboxylate salt may generate an acid upon light exposure and may serve as a quenching base to neutralize an acid before light exposure. Accordingly, the carboxylate salt may be used in combination with a photoacid generator that generates an acid. In addition, since the carboxylate salt may generate an acid upon light exposure, quencher functions of the carboxylate salt may be lost by neutralization with the acid generated by the carboxylate salt, and thus the contrast between the exposed area and the unexposed area may be further enhanced.

The carboxylate salt may be used in an amount in a range of about 0.1 parts by weight to about 40 parts by weight (for example, about 5 parts by weight to about 30 parts by weight) based on 100 parts by weight of the base resin. When the amount of the carboxylate salt is within these ranges, the carboxylate salt may exhibit quencher functions at an appropriate level, and formation of foreign particles may be reduced due to loss of any performance, e.g., a decrease in sensitivity and/or a lack of solubility.

The carboxylate salt is the same as described above, and thus the organic solvent, the base resin, the photoacid generator, and optional components included as necessary will be described below. In addition, for use as the carboxylate salt represented by Formula 1 in the photoresist composition, one type of the carboxylate salt or a combination of two or more different types of the carboxylate salt may be used.

### <Organic solvent>

The organic solvent included in the photoresist composition is not particularly limited as long as it is capable of dissolving and/or dispersing the carboxylate salt, the base resin, the photoacid generator, and optional components contained as necessary. One type of organic solvent may be used, and/or a combination of two or more different types of the organic solvent may be used. Also, a mixed solvent in which water and an organic solvent are mixed may be used.

Examples of types of organic solvents are alcohol-based solvents, ether-based solvents, ketone-based solvents, amide-based solvents, ester-based solvents, sulfoxide-based solvents, hydrocarbon-based solvents, and/or the like.

For example, examples of the alcohol-based solvent include a monoalcohol-based solvent (such as methanol, ethanol, n-propanol, isopropanol, 1-methoxy-2-propanol, 1-ethoxy-2-propanol, n-butanol, isobutanol, sec-butanol, tert-butanol, n-pentanol, isopentanol, 2-methylbutanol, sec-pentanol, tert-pentanol, 3-methoxybutanol, 3-methyl-3-methoxybutanol, n-hexanol, 2 -methylpentanol, sec-hexanol, 2-ethylbutanol, sec-heptanol, 3-heptanol, n-octanol, 2-ethylhexanol, sec-octanol, n-nonyl alcohol, 2,6-dimethyl-4-heptanol, n-decanol, sec-undecyl alcohol, trimethylnonyl alcohol, sec-tetradecyl alcohol, sec-heptadecyl alcohol, furfuryl alcohol, phenol, cyclohexanol, methylcyclohexanol, 3,3,5-trimethylcyclohexanol, benzyl alcohol, diacetone alcohol, and/or the like); a polyhydric alcohol-based solvent (such as ethylene glycol, 1,2-propylene glycol, 1,3-butylene glycol, 2,4-pentanediol, 2-methyl-2,4-pentanediol, 2,5-hexanediol, 2,4-heptanediol, 2-ethyl-1,3-hexanediol, diethylene glycol, dipropylene glycol, triethylene glycol, tripropylene glycol, and/or the like); a polyhydric alcohol-containing ether-based solvent (such as ethyleneglycol monomethyl ether, ethylene glycol monoethyl ether, ethylene glycol monopropyl ether, ethylene glycol monobutyl ether, ethylene glycol monohexyl ether, ethylene glycol monophenyl ether, ethylene glycol mono-2-ethylbutyl ether, diethylene glycol monomethyl ether, diethylene glycol monoethyl ether, diethylene glycol monopropyl ether, diethylene glycol monobutyl ether, diethylene glycol monohexyl ether, diethylene glycol dimethyl ether, propylene glycol monomethyl ether, propylene glycol dimethyl ether, propylene glycol monoethyl ether, propylene glycol monopropyl ether, propylene glycol monobutyl ether, dipropylene glycol monomethyl ether, dipropylene glycol monoethyl ether, dipropylene glycol monopropyl ether, and/or the like); and/or the like.

Examples of the ether-based solvent includes a dialkyl ether-based solvent (such as diethyl ether, dipropyl ether, dibutyl ether, and/or the like); a cyclic ether-based solvent (such as tetrahydrofuran, tetrahydropyran, and/or the like); an aromatic ring-containing ether-based solvent (such as diphenyl ether, anisole, and/or the like); and/or the like.

Examples of the ketone-based solvent include a chain ketone-based solvent (such as acetone, methylethyl ketone, methyl-n-propyl ketone, methyl-n-butyl ketone, methyl-n-pentyl ketone, diethyl ketone, methyl isobutyl ketone, 2-heptanone, ethyl-n-butyl ketone, methyl-n-hexyl ketone, diisobutyl ketone, trimethylnonanone, and/or the like; a cyclic ketone solvent, such as cyclopentanone, cyclohexanone, cycloheptanone, cyclooctanone, methylcyclohexanone, and/or the like); 2,4-pentanedione; acetonylacetone; acetphenone; and/or the like.

Examples of the amide-based solvent include a cyclic amide-based solvent (such as N,N'-dimethylimidazolidinone, N-methyl-2-pyrrolidone, and/or the like); a chain amide-based solvent (such as N-methylformamide, N,N-dimethylformamide, N,N-diethylformamide, acetamide, N-methylacetamide, N,N-dimethylacetamide, N-methylpropionamide, and/or the like); and/or the like.

Examples of the ester-based solvent are: an acetate ester-based solvent (such as methyl acetate, ethyl acetate, n-propyl acetate, isopropyl acetate, n-butyl acetate, isobutyl acetate, sec-butyl acetate, t-butyl acetate, n-pentyl acetate, isopentyl acetate, sec-pentyl acetate, 3-methoxybutyl acetate, methylpentyl acetate, 2-ethylbutyl acetate, 2-ethylhexyl acetate, benzyl acetate, cyclohexyl acetate, methylcyclohexyl acetate, n-nonyl acetate, and/or the like; a polyhydric alcohol-containing ether carboxylate-based solvent (such as ethylene glycol monomethyl ether acetate, ethylene glycol monoethyl ether acetate, diethylene glycol monomethyl ether acetate, diethylene glycol monoethyl ether acetate, diethylene glycol mono-n-butyl ether acetate, propylene glycol monomethyl ether acetate, propylene glycol monoethyl ether acetate, propylene glycol monopropyl ether acetate, propylene glycol monobutyl ether acetate, dipropylene glycol monomethyl ether acetate, dipropylene glycol monoethyl ether acetate, and/or the like); a lactone-based solvent (such as γ-butylolactone, δ-valerolactone, and/or the like); a carbonate-based solvent (such as dimethyl carbonate, diethyl carbonate, ethylene carbonate, propylene carbonate, and/or the like); a lactate ester-based solvent (such as methyl lactate, ethyl lactate, n-butyl lactate, n-amyl lactate, and/or the like); glycoldiacetate, methoxytriglycol acetate, propionic acid ethyl, propionic acid n-butyl, propionic acid isoamyl, diethyloxalate, di-n-butyloxalate, methyl acetoacetate, ethyl acetoacetate, malonic acid diethyl, phthalic acid dimethyl, phthalic diethyl, and/or the like.

Examples of the sulfoxide-based solvent include dimethyl sulfoxide, diethyl sulfoxide, and/or the like.

Examples of the hydrocarbon-based solvent include an aliphatic hydrocarbon-based solvent (such as n-pentane, isopentane, n-hexane, isohexane, n-heptane, isoheptane, 2,2,4-trimethyl pentane, n-octane, isooctane, cyclohexane, methylcyclohexane, and/or the like); an aromatic hydrocarbon-based solvent (such as benzene, toluene, xylene, mesitylene, ethyl benzene, trimethyl benzene, methylethyl benzene, n-propyl benzene, isopropyl benzene, diethyl benzene, isobutyl benzene, triethyl benzene, diisopropyl benzene, n-amylnaphthalene, and/or the like); and/or the like.

In one or more embodiments, the organic solvent may be selected from an alcohol-based solvent, an amide-based solvent, an ester-based solvent, a sulfoxide-based solvent, and a combination thereof. In one or more embodiments, the organic solvent may be selected from propylene glycol monomethyl ether, propylene glycol monoethyl ether, propylene glycol monomethyl ether acetate, N-methyl-2-pyrrolidone, N,N-dimethylacetamide, ethyl lactate, dimethyl sulfoxide, and a combination thereof.

In one or more embodiments, when an acetal-type acid labile group is used, the organic solvent may further include alcohol having a high boiling point (such as diethylene glycol, propylene glycol, glycerol, 1,4-butanediol, 1,3-butanediol, and/or the like), to accelerate a deprotection reaction of acetal.

The organic solvent may be used in an amount in a range of about 200 parts by weight to about 5,000 parts by weight, for example, about 400 parts by weight to about 3,000 parts by weight, based on 100 parts by weight of the base resin.

### <Base resin>

The base resin may include a repeating unit represented by Formula 4 and including an acid labile group:

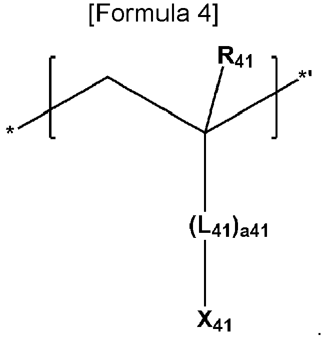

In Formula 4, R₄₁ may be at least one of hydrogen, deuterium, a halogen, a linear or branched C₁-C₆ alkyl group, and/or a linear or branched C₁-C₆ halogenated alkyl group; L₄₁ may be at least one of a single bond, a substituted or unsubstituted C₁-C₁₀ alkylene group, a substituted or unsubstituted C₃-C₁₀ cycloalkylene group, a substituted or unsubstituted C₃-C₁₀ heterocycloalkylene group, a substituted or unsubstituted phenylene group, a substituted or unsubstituted naphthylene group, *-O-*', *-C(=O)O-*', -OC(=O)-*', *-C(=O)NH-*', -NHC(=O)-*', or a combination thereof; a41 may be an integer from 1 to 6; X₄₁ may be an acid labile group; and * and *' each indicate a binding site to a neighboring atom.

For example, in one or more embodiments, in Formula 4, R₄₁ may be hydrogen, deuterium, F, CH₃, CH₂F, CHF₂, and/or CF₃.

When L₁₁ in Formula 4 is a "C₁-C₁₀ alkylene group", examples of the C₁-C₁₀ alkylene group include a methylene group, an ethylene group, a propylene group, a butylene group, an isobutylene group, and/or the like.

When L₁₁ in Formula 4 is a "C₃-C₁₀ cycloalkylene group", examples of the C₃-C₁₀ cycloalkylene group include a cyclopentylene group, a cyclohexylene group, an adamantylene group, an adamantylmethylene group, a norbornylene group, a norbornylmethylene group, a tricyclodecanylene group, a tetracyclododecanylene group, a tetracyclododecanylmethylene group, a dicyclohexylmethylene group, and/or the like.

When L₁₁ in Formula 4 is a "C₁-C₁₀ heterocycloalkylene group", the C₁-C₁₀ heterocycloalkylene group may refer to a group in which some carbon atoms of the "C₃-C₁₀ cycloalkylene group" are substituted with a moiety including a heteroatom (such as oxygen, sulfur, nitrogen, etc.). In this regard, the "C₁-C₁₀ heterocycloalkylene group" may include an ether linkage, an ester linkage, a sulfonic ester linkage, a carbonate, a lactone ring, a sultone ring, or a carboxylic anhydride moiety.

In Formula 4, a41 indicates the number of repetitions of L₁₁, wherein, when a41 is 2 or more, a plurality of L₁₁ may be identical to or different from each other.

In one or more embodiments, X₄₁ in Formula 4 may be represented by at least one of Formulae 6-1 to 6-7:

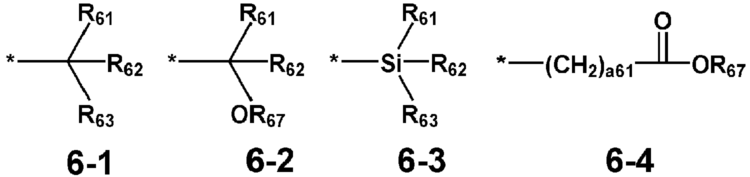

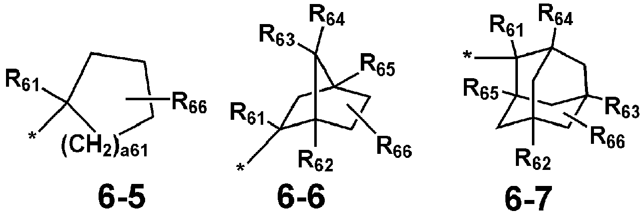

In Formulae 6-1 to 6-7, a61 may be an integer from 0 to 6, R₆₁ to R₆₆ may each independently be at least one of hydrogen or a linear, branched, or cyclic C₁-C₂₀ monovalent hydrocarbon group that optionally includes a heteroatom, R₆₇ may be a linear, branched, or cyclic C₁-C₂₀ monovalent hydrocarbon group that optionally includes a heteroatom, two adjacent groups among R₆₁ to R₆₇ may optionally be bonded to each other to form a ring, and * indicates a binding site to a neighboring atom.

In Formulae 6-4 and 6-5, (CH₂)ₐ₆₁ may be a single bond when a61 is 0.

The "monovalent hydrocarbon group" of R₆₁ to R₆₇ in Formulae 6-1 to 6-7 may be the same as defined in connection with the "monovalent hydrocarbon group" of R₁₁ in Formula 1.

In one or more embodiments, the repeating unit represented by Formula 4 may be represented by at least one of Formulae 4-1 and 4-2:

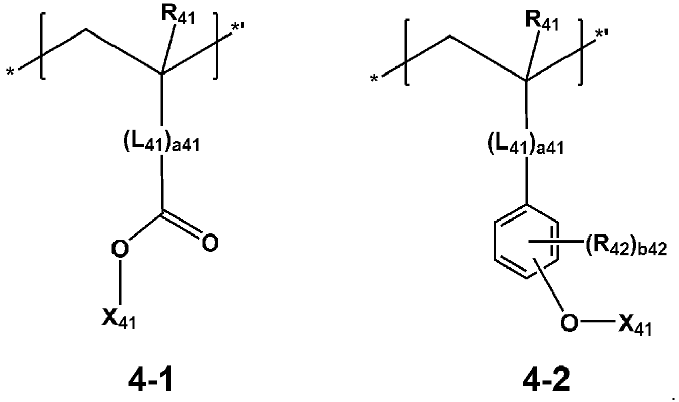

In Formulae 4-1 and 4-2, L₄₁ and X₄₁ may each be the same as defined in Formula 4, a41 may be an integer from 1 to 4, R₄₂ may be hydrogen (e.g., protium) or a linear, branched, or cyclic C₁-C₂₀ monovalent hydrocarbon group that optionally includes a heteroatom, b42 may be an integer from 1 to 4, and * and *' each indicate a binding site to a neighboring atom.

The "monovalent hydrocarbon group" of R₄₂ in Formula 4-2 may be the same as defined in connection with the "monovalent hydrocarbon group" of R₁₁ in Formula 1.

The base resin including the repeating unit represented by Formula 4 may be decomposed under the action of an acid to generate a carboxyl group, and thus may be converted to have alkali solubility.

The base resin may further include, in addition to the repeating unit represented by Formula 4, a second repeating unit represented by Formula 5:

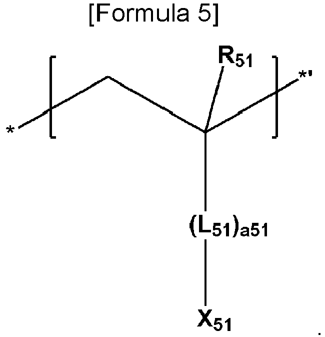

In Formula 5, R₅₁ may be at least one of hydrogen, deuterium, a halogen, a linear or branched C₁-C₆ alkyl group, or a linear or branched C₁-C₆ halogenated alkyl group; L₅₁ may be at least one of a single bond, a substituted or unsubstituted C₁-C₁₀ alkylene group, a substituted or unsubstituted C₃-C₁₀ cycloalkylene group, a substituted or unsubstituted C₃-C₁₀ heterocycloalkylene group, a substituted or unsubstituted phenylene group, a substituted or unsubstituted naphthylene group, *-O-*', *-C(=O)O-*', -OC(=O)-*', *-C(=O)NH-*', -NHC(=O)-*', or a combination thereof; a51 may be an integer from 1 to 6; X₅₁ may be a non-acid labile group; and * and *' each indicate a binding site to a neighboring atom.

For example, in Formula 5, R₅₁ may be the same as defined in connection with R₄₁ in Formula 4; L₅₁ may be the same as defined in connection with L₄₁ in Formula 4; and/or a51 indicates the number of repetitions of L₅₁, wherein, when a51 is 2 or more, a plurality of L₅₁ may be identical to or different from each other.

In one or more embodiments, X₅₁ in Formula 5 may be at least one of hydrogen (e.g., protium); or a linear, branched, or cyclic C₁-C₂₀ monovalent hydrocarbon group that includes one or more polar moieties selected from a hydroxyl group, a halogen, a cyano group, a carbonyl group, a carboxyl group, *-O-*', *-C(=O)O-*', -OC(=O)-*', *-S(=O)O-*', -OS(=O)-*', a lactone ring, a sultone ring, and/or a carboxylic anhydride moiety. Here, the "monovalent hydrocarbon group" may be the same as defined in connection with the "monovalent hydrocarbon group" among the examples of R₁₁ in Formula 1, may essentially include one or more polar moieties selected from a hydroxyl group, halogen, a cyano group, a carbonyl group, a carboxyl group, *-O-*', *-C(=O)O-*', -OC(=O)-*', *-S(=O)O-*', -OS(=O)-*', a lactone ring, a sultone ring, and a carboxylic anhydride moiety.

In one or more embodiments, the repeating unit represented by Formula 5 may be represented by at least one of Formulae 5-1 and 5-2:

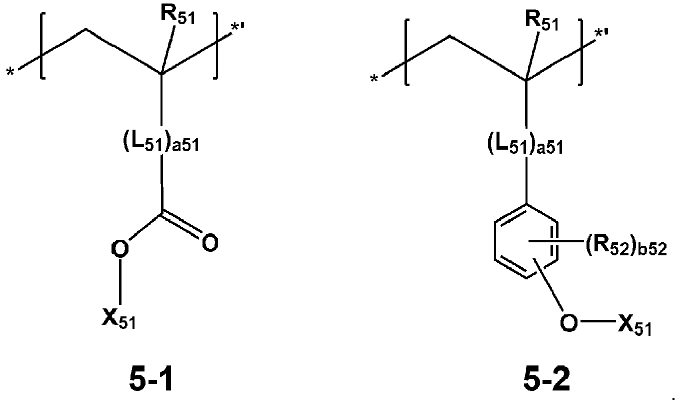

In Formulae 5-1 and 5-2, L₅₁ and X₅₁ may each be as defined in Formula 5; a51 may be an integer from 1 to 4; R₅₂ may be at least one of hydrogen or a hydroxyl group; and/or a linear, branched, or cyclic C₁-C₂₀ monovalent hydrocarbon group that optionally includes a heteroatom; b52 may be an integer from 1 to 4, and * and *' each indicate a binding site to a neighboring atom.

The "monovalent hydrocarbon group" of R₅₂ in Formula 5-2 may be the same as defined in connection with the "monovalent hydrocarbon group" of R₁₁ in Formula 1.

For example, in an Argon Fluoride (ArF) lithography process, X₅₁ may include a lactone ring as a polar moiety, and/or in Krypton Fluoride (KrF), electron beam (EB), and/or extra ultraviolet (EUV) lithography processes, X₅₁ may be phenol.

In one or more embodiments, the base resin may further include a moiety including an anion and/or a cation. For example, the base resin may further include a moiety in which a photoacid generator and/or a quencher are induced to bind to the side chain.

The base resin may have a weight average molecular weight (Mw) in a range of about 1,000 to 500,000, for example, about 3,000 to about 100,000, wherein the weight average molecular weight (Mw) is measured by gel permeation chromatography using a tetrahydrofuran solvent and polystyrene as a standard material.

The base resin may have polydispersity index (PDI, Mw/Mn) in a range of about 1.0 to about 3.0, for example, about 1.0 to about 2.0. When the PDI of the base resin is satisfied within the ranges above, there is a less chance of remaining foreign substances on a pattern, or deterioration of a pattern profile may be minimized. Accordingly, the photoresist composition may become more suitable for forming a fine pattern.

The base resin may be prepared by any suitable method. For example, the base resin may be prepared by dissolving monomer(s) including unsaturated linkages in an organic solvent, followed by performing thermal polymerization in the presence of a radical initiator.

In the base resin, a mole fraction (mol%) of each repeating unit derived from each monomer is as follows, but is not limited thereto:
i) the repeating unit represented by Formula 4 is included in an amount in a range of about 1 mol% to about 60 mol%, for example, about 5 mol% to about 50 mol%, and for example, about 10 mol% to about 50 mol%; and
ii) the repeating unit represented by Formula 5 is included in an amount in a range of about 40 mol% to about 99 mol%, for example, about 50 mol% to about 95 mol%, and for example, about 50 mol% to about 90 mol%.

The base resin may be a homopolymer and/or may include a mixture of two or more types of polymers having a different composition, weight average molecular weight (Mw), and/or PDI (Mw/Mn).

### <Photoacid generator>

The photoacid generator may include any compound capable of generating an acid upon exposure to high-energy rays (such as UV, deep UV (DUV), EB, EUV, X-ray, excimer laser, γ-ray, and/or the like).

The photoacid generator may include at least one of a sulfonium salt, an iodonium salt, and/or a combination thereof.

In one or more embodiments, the photoacid generator may be represented by Formula 7:

[Formula 7] B₇₁⁺ A₇₁⁻.

In Formula 7, B₇₁⁺ may be represented by Formula 7A, and A₇₁⁻ may be represented by one of Formulae 7B to 7D, and B₇₁⁺ and A₇₁⁻ may be linked via at least one of an ionic bond (e.g., a charged based interaction) and/or a carbon-carbon covalent bond (e.g., an electron sharing interaction between carbon atoms).

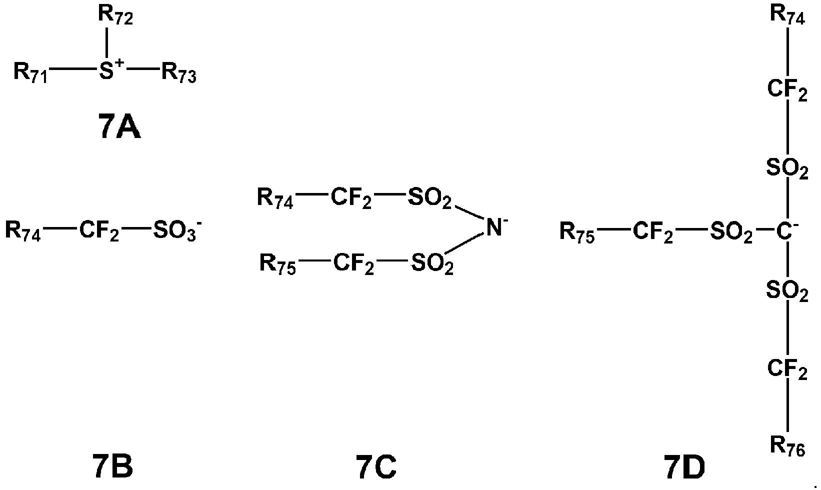

In Formulae 7A to 7D, R₇₁ to R₇₃ may each independently be a linear, branched, or cyclic C₁-C₂₀ monovalent hydrocarbon group; two adjacent groups among R₇₁ to R₇₃ may optionally be bonded to each other to form a ring; and R₇₄ to R₇₆ are each independently at least one of fluorene (F); and/or a linear, branched, or cyclic C₁-C₂₀ monovalent hydrocarbon group that optionally includes a heteroatom.

In Formula 7A, R₇₁ to R₇₃ may each be the same as defined in connection with R₃₁ to R₃₅ in Formula 3-1.

Regarding the description of R₇₄ to R₇₆ in Formulae 7B to 7D, the monovalent hydrocarbon may include, for example, a linear or branched alkyl group (e.g., a methyl group, an ethyl group, a propyl group, an isopropyl group, a butyl group, an isobutyl group, a sec-butyl group, a tert-butyl group, a pentyl group, a neopentyl group, a hexyl group, a heptyl group, a 2-ethylhexyl group, a nonyl group, an undecyl group, a tridecyl group, a pentadecyl group, a heptadecyl group, an eicosanyl group, and/or the like); a monovalent saturated alicyclic hydrocarbon group (e.g., a cyclopentyl group, a cyclohexyl group, a 1-adamantyl group, a 2-adamantyl group, a 1-adamantylmethyl group, a norbornyl group, a norbornylmethyl group, a tricyclotricyclodecanyl group, a tetracyclododecanyl group, a tetracyclododecanylmethyl group, a dicyclohexylmethyl group and/or the like); a monovalent unsaturated aliphatic hydrocarbon group (e.g., an allyl group, a 3-cyclohexenyl group, and/or the like); an aryl group (e.g., a phenyl group, a 1-naphthyl group, a 2-naphthyl group, and/or the like); an arylalkyl group (e.g., a benzyl group, a diphenylmethyl group, and/or the like); a heteroatom-containing monovalent hydrocarbon group (e.g., a tetrahydrofuranyl group, a methoxymethyl group, an ethoxymethyl group, a methylthiomethyl group, an acetamidemethyl group, a trifluoroethyl group, a (2-methoxyethoxy)methyl group, an acetoxymethyl group, a 2-carboxy-1-cyclohexyl group, a 2-oxopropyl group, a 4-oxo-1-adamantyl group, a 3-oxocyclohexyl group, and/or the like), and/or the like. In addition, among these groups, some hydrogen atoms may be substituted with a moiety including a heteroatom (such as oxygen, sulfur, nitrogen, or a halogen atom), and/or some carbon atoms may be replaced by a moiety including a heteroatom (such as oxygen, sulfur, or nitrogen). Accordingly, these groups may each include a hydroxy group, a cyano group, a carbonyl group, a carboxyl group, an ether linkage, an ester linkage, a sulfonic ester linkage, a carbonate, a lactone ring, a sultone ring, a carboxylic anhydride moiety, a haloalkyl moiety, and/or the like.

For example, in Formula 7, B₇₁⁺ may be represented by Formula 7A, and A₇₁⁻may be represented by Formula 7B. In detail, R₇₁ to R₇₃ in Formula 7A may each be a phenyl group, and R₇₄ in Formula 7B may be a propyl group substituted with F.

The photoacid generator may be included in an amount in a range of about 0 parts by weight to about 40 parts by weight, about 0.1 parts by weight to about 40 parts by weight, or about 0.1 parts by weight to about 20 parts by weight, based on 100 parts by weight of the base resin. When the amount of the photoacid generator is satisfied within the ranges above, proper resolution may be achieved, and problems related to foreign particles after development or during stripping may be reduced.

One type of the photoacid generator may be used, or a combination of two or more different types of the photoacid generator may be used.

### <Optional components>

The photoresist composition may further include a surfactant, a cross-linking agent, a leveling agent, a colorant, or any combination thereof, as needed.

The photoresist composition may further include a surfactant to improve coating properties, developability, and/or the like. Examples of the surfactant include a non-ionic surfactant (such as polyoxyethylene lauryl ether, polyoxyethylene stearyl ether, polyoxyethylene oleyl ether, polyoxyethylene n-octylphenyl ether, polyoxyethylene n-nonylphenyl ether, polyethylene glycol dilaurate, polyethylene glycol distearate, and/or the like); and/or the like. For use as the surfactant, a commercially available product may be used, and/or a synthesized product may be used. Examples of the commercially available product are KP341 (manufactured by Shin-Etsu Chemical Co., Ltd.), POLYFLOW No.75 and POLYFLOW No.95 (manufactured by Kyoeisha Chemical Co., Ltd.), FTOP EF301, FTOP EF303, and FTOP EF352 (manufactured by Mitsubishi Materials Electronic Chemicals Co., Ltd.), MEGAFACE F171 (registered trademark), MEGAFACE F173, R40, R41, and R43 (manufactured by DIC Corporation), Fluorad FC430 (registered trademark) and Fluorad FC431 (manufactured by 3M Company), AsahiGuard AG710 (product of AGC Corporation), Surflon S-382 (registered trademark), Surflon SC-101, Surflon SC-102, Surflon SC-103, Surflon SC-104, Surflon SC-105, Surflon SC-106 (manufactured by AGC Seimi Chemical Co., Ltd.), and/or the like.

The surfactant may be included in an amount in a range of about 0 parts by weight to about 20 parts by weight based on 100 parts by weight of the base resin. One type of the surfactant may be used, or a combination of two or more different types of the surfactant may be used.

A method of preparing the photoresist composition is not particularly limited. For example, a method of mixing the carboxylate salt, the base resin, the photoacid generator, and optional components added as necessary in the organic solvent may be used. The temperature or time at the mixing is not particularly limited. Filtration may be performed after the mixing as needed.

### [Pattern forming method]

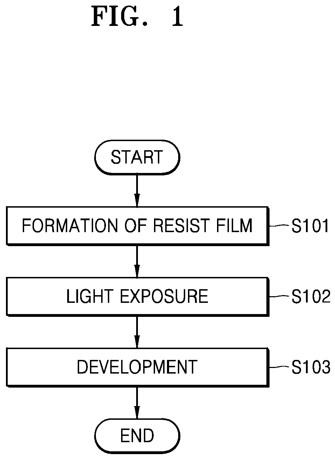

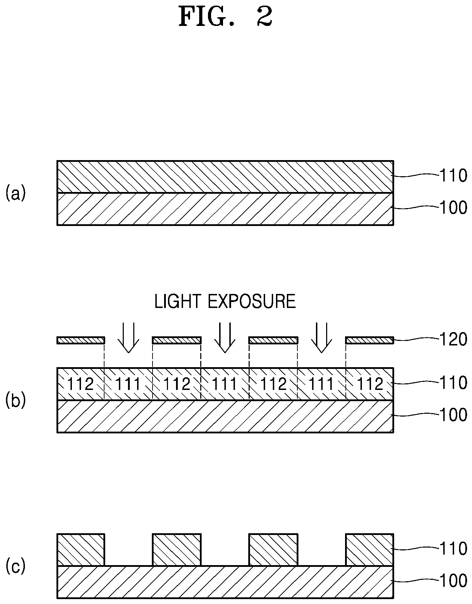

Hereinafter, a method of forming a pattern according to one or more embodiments will be described in detail with reference to FIGS. 1 and 2. FIG. 1 is a flowchart representing a pattern forming method according to one or more embodiments, and FIG. 2 is a side cross-sectional view illustrating a pattern forming method according to one or more embodiments. Hereinafter, a method of forming a pattern by using a positive photoresist composition will be described in detail as an example, but embodiments are not limited thereto.

Referring to FIG. 1, a method of forming a pattern includes forming a photoresist film by applying a photoresist composition (S101); exposing at least a portion of the photoresist film with high-energy rays (S102); and developing the exposed photoresist film by using a developing solution (S103). The steps above may be omitted as necessary, and/or may be performed in different orders.

First, a board 100 is prepared. The board 100 may be, for example, a semiconductor board (such as a silicon board or a germanium board), and/or may be formed of glass, quartz, ceramic, copper, and/or the like. In one or more embodiments, the board 100 may include a Group III-V compound (such as GaP, GaAs, GaSb, and/or the like), and/or the like.

A photoresist film 110 may be formed by coating the board 100 with a photoresist composition to a desired thickness according to a coating method. As needed, a heating process may be performed thereon to remove an organic solvent remaining in the photoresist film 110. The coating method may include spin coating, dipping, roller coating, and/or other common coating methods. Among these methods, spin coating may be particularly used, and the photoresist film 110 having a desired thickness may be formed by adjusting the viscosity, concentration, and/or spin speed of the photoresist composition. In one or more embodiments, a thickness of the photoresist film 110 may be in a range of about 10 nm to about 300 nm. In one or more embodiments, a thickness of the photoresist film 110 may be in a range of about 30 nm to about 200 nm.

The lower limit of a pre-baking temperature may be 60 °C or higher, for example, 80 °C or higher. In addition, the upper limit of a pre-baking temperature may be 150 °C or lower, for example, 140 °C or lower. The lower limit of a pre-baking time may be 5 seconds or more, for example, 10 seconds or more. The upper limit of a pre-baking time may be 600 seconds or less, for example, 300 seconds or less. In at least some embodiments, excessive heat and/or baking may result in deterioration of pattern precision due to, e.g., degradation of the photoresist composition.

Before coating the board 100 with the photoresist composition, a film to be etched (not shown) may be further formed on the board 100. The film to be etched may refer to a layer in which an image is transferred from a photoresist pattern and converted into a predetermined pattern. In one or more embodiments, the film to be etched may be formed to include, for example, an insulating material (such as silicon oxide, silicon nitride, or silicon oxynitride). In one or more embodiments, the film to be etched may be formed to include a conductive material (such as metal, metal nitride, metal silicide, or metal silicide nitride). In one or more embodiments, the film to be etched may be formed to include a semiconductor material (such as polysilicon).

In one or more embodiments, an antireflection layer may be further formed on the board 100 to exhibit efficiency of the photoresist at most. The antireflection layer may include an organic-based antireflection layer and/or an inorganic-based antireflection layer.

In one or more embodiments, a protective layer may be further provided on the photoresist film 110 to reduce the influence of alkaline impurities included in the process. In addition, when performing immersion exposure, for example, a protective film against immersion may be provided on the photoresist film 110 to avoid direct contact between an immersion medium and the photoresist film 110.

Next, at least a portion of the photoresist film 110 may be exposed with high-energy rays. For example, high-energy rays passing through a mask 120 may be irradiated to at least a portion of the photoresist film 110. Accordingly, the photoresist film 110 may have an exposed area 111 and an unexposed area 112.

The exposure may be carried out by irradiating high-energy rays through a mask having a predetermined (or otherwise determined) pattern and by using a liquid (such as water and/or the like), as a medium in some cases. Examples of the high-energy rays are electromagnetic waves (such as ultraviolet (UV) rays, far-ultraviolet rays, extreme ultraviolet rays (EUV rays, wavelength of 13.5 nm), X-rays, γ-rays, and/or the like); charged particle beams (such as electron beams (EBs), α rays, and/or the like); and/or the like. The irradiation of such high-energy rays may be collectively referred to as "exposure".

For use as a light source of the exposure, various types of irradiation including irradiating laser beams in the ultraviolet region (such as KrF excimer laser (wavelength of 248 nm), ArF excimer laser (wavelength of 193 nm), and an F₂ excimer laser (wavelength of 157 nm), irradiating harmonic laser beams in the far ultraviolet or vacuum ultraviolet region by a wavelength conversion method using laser beams from a solid-state laser source (e.g., YAG or semiconductor laser), irradiating electron beams or EUV rays, and/or the like may be used. Upon the exposure, the exposure may be performed through a mask corresponding to a desired pattern. However, when the light source of the exposure is electron beams, the exposure may be performed by direct drawing without using a mask.

The integral dose of the high-energy rays may be less than or equal to 2,000 mJ/cm², for example, less than or equal to 500 mJ/cm², in the case of using EUV rays as the high-energy rays. In addition, in the case of using electron beams as the high-energy rays, the integral dose of the high-energy rays may be 5,000 µC/cm² or less, for example, 1,000 µC/cm² or less.

In addition, post-exposure baking (PEB) may be performed after the exposure. The lower limit of a PEB temperature may be 50 °C or higher, for example, 80 °C or higher. The upper limit of the PEB temperature may be 180 °C or less, for example, 130 °C or less. The lower limit of a PEB time may be 5 seconds or more, for example, 10 seconds or more. The upper limit of a PEB time may be 600 seconds or less, for example, 300 seconds or less.

Next, the exposed photoresist film 110 may be developed by using a developing solution. For example, in the case wherein the photoresist composition is a positive composition, the exposed area 111 may be washed away by the developing solution, whereas the unexposed area 112 may remain without being washed away by the developing solution.

For use as the developing solution, an alkali developing solution, a developing solution containing an organic solvent (hereinafter also referred to as "organic developing solution"), and/or the like may be used. As a developing method, a dipping method, a puddle method, a spray method, a dynamic administration method, and/or the like may be used. The developing temperature may be, for example, about 5 °C or more and about 60°C or less, and the developing time may be, for example, about 5 seconds or more and about 300 seconds or less.

The alkali developing solution may be, for example, an alkaline aqueous solution which dissolves at least one alkaline compound (such as sodium hydroxide, potassium hydroxide, sodium carbonate, sodium silicate, sodium metasilicate, aqueous ammonia, ethylamine, n-propylamine, diethylamine, di-n-propylamine, triethylamine, methyldiethylamine, ethyldimethylamine, triethanolamine, tetramethyl ammonium hydroxide (TMAH), pyrrole, piperidine, choline, 1,8-diazabicyclo[5.4.0]-7-undecene (DBU), 1,5-diazabicyclo[4.3.0]-5-nonene, and/or the like). The alkaline developing solution may further include a surfactant.

The lower limit of the amount of the alkaline compound in the alkaline developing solution may be 0.1 mass% or more, for example, 0.5 mass% or more, and/or for example, 1 mass% or more. In addition, the upper limit of the amount of the alkaline compound in the alkaline developing solution may be 20 mass% or less, for example, 10 mass% or less, and for example, 5 mass% or less.

After the development, a resulting photoresist pattern may be washed with ultrapure water, and subsequently, the water remaining on the board 100 and the pattern may be removed.

As an organic solvent contained in the organic developing solution, for example, the same organic solvent as the organic solvent described in the <Organic solvent> of the [Resist composition] may be used.

The lower limit of the amount of the organic solvent in the organic developing solution may be 80 mass % or more, for example, 90 mass % or more, and for example, 95 mass % or more, and for example, 99 mass % or more.

The organic developing solution may also include a surfactant. In addition, the organic developing solution may include a trace amount of moisture. In addition, upon the development, the solvent may be substituted with a solvent of a different kind from the organic developing solution to stop the development.

The photoresist pattern resulting from the development may be further washed. As a washing solution, ultrapure water, a liquid rinse, and/or the like may be used. The liquid rinse is not particularly limited as long as it does not dissolve the exposed photoresist pattern, and a solution containing a general organic solvent may be used. For example, the liquid rinse may be an alcohol-based solvent or an ester-based solvent. After the washing, the liquid rinse remaining on the board and the pattern may be removed. In addition, when ultrapure water is used, the water remaining on the board and the pattern may be removed.

In addition, the developing solution may be used either individually or in a combination of two or more.

After the photoresist pattern is formed as described above, an etching process may be performed thereon to obtain a patterned wiring board. The etching method may be performed by known methods including dry etching using plasma gas; wet etching using an alkali solution, a cupric chloride solution, a ferric chloride solution; and/or the like.

After forming the resist pattern, plating may be performed. Although not particularly limited, the plating method may include, for example, copper plating, solder plating, nickel plating, gold plating, and/or the like.

The photoresist pattern remaining after the etching may be exfoliated with an organic solvent. Although not particularly limited, examples of the organic solvent are propylene glycol monomethyl ether acetate (PGMEA) propylene glycol monomethyl ether (PGME), ethyl lactate (EL), and/or the like. Although not particularly limited, examples of the exfoliation method are an immersion method, a spray method, and/or the like. In addition, the wiring board on which the photoresist pattern is formed may be a multilayer wiring board, and may have through holes with a small diameter.

In one or more embodiments, after the formation of the photoresist pattern, the wiring board may be formed by a so-called lift-off process in which metal is deposited in a vacuum and then the photoresist pattern is dissolved in a solution.

The disclosure will be described in more detail with reference to Examples and Comparative Examples below, but the technical scope of the disclosure is not limited only thereto.

### [Examples]

### Synthesis Example 1: Synthesis of PDQ A

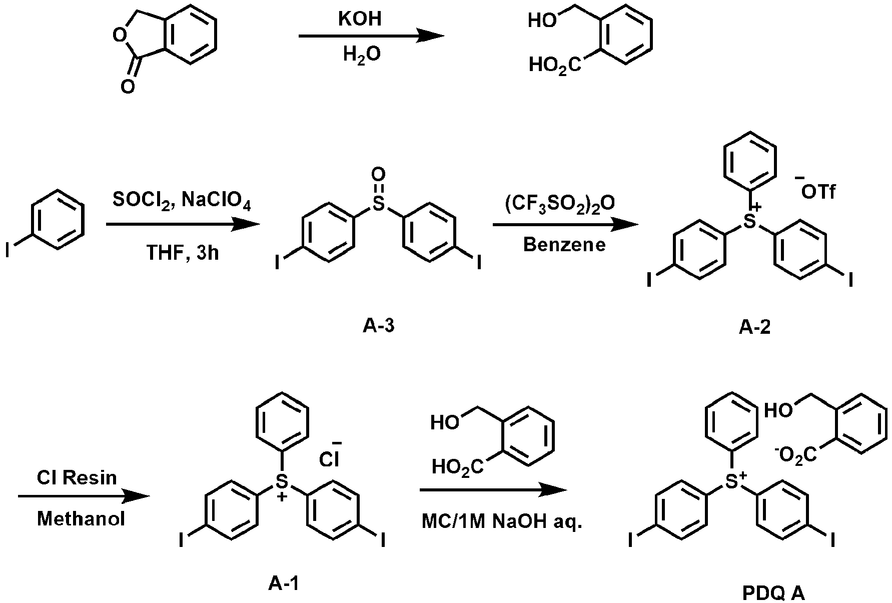

### (Anion part)

Phthalide (2.68 g, 20 mmol, 1 eq.) and potassium hydroxide (KOH) (1.68 g, 30 mmol, 1.5 eq.) were mixed in 150 mL of 85 % methanol (methanol: water = 125 mL: 25 mL, v/v), and stirred for 90 minutes under reflux by heating. The resultant mixture was cooled to room temperature, and then precipitated with hydrochloric acid. After filtering the precipitate, the filtrate was washed with cold water and dried under vacuum. A compound thus obtained was identified by ¹H-NMR and MS.

¹H-NMR (500MHz, DMSO-d 6) δ 7.85 (d, 1H), 7.70 (d, 1H), 7.55 (t, 1H), 7.32 (t, 1H), 4.86 (s, 2H).

MS (ESI -) m/z 151.0480.

### (Cation part)

### 1) Synthesis of Compound A-3

lodobenzene (2.246 g, 11.01 mmol), thionyl chloride (0.655 g, 5.51 mmol), and sodium perchlorate (0.117 g, 1.10 mmol) were mixed in 12 mL of tetrahydrofuran, and stirred for 3 hours. Next, after removing the reaction solvent by distillation under reduced pressure, an organic layer obtained by extraction using 30 mL of water and 30 mL of methylene chloride was dried using Na₂SO₄, and then filtered. The filtrate thus obtained was decompressed, and the resultant residue was separated and purified by silica gel column chromatography to obtain Compound A-3 (3.75 g, 75 %).

### 2) Synthesis of Compound A-2

Compound A-3 (3.73 g, 8.20 mmol) was dissolved in 15 mL of benzene, and trifluoromethanesulfonic anhydride (2.778 g, 9.85 mmol) was added dropwise thereto at 0 °C, and the resultant solution was stirred for 1 hour at room temperature. Next, an organic layer obtained by extraction using 20 mL of water and 50 mL of ethyl acetate was washed using saturated NaHCOs aqueous solution, dried using MgSO₄, and then filtered. The filtrate thus obtained was decompressed, and the resultant residue was separated and purified by silica gel column chromatography to obtain Compound A-2 (4.92 g, 90 %).

### 3) Synthesis of Compound A-1

Compound A-2 (2 g, 3.01 mmol) and Cl resin (10 g) were mixed in 10 mL of methanol, and stirred for 2 hours. Next, the resultant solution was filtered, and the filtrate was distilled under reduced pressure to obtain Compound A-1 (1.59 g, 96 %).

### (Synthesis of Compound PDQ A)

Compound A-1 (0.5 g, 0.91 mmol) and 2-(hydroxymethyl)benzoic acid (0.14 g, 0.91 mmol) were mixed in 5 mL of methylene chloride and 5 mL of 1 M NaOH aqueous solution, and stirred for 2 hours. Next, an organic layer obtained by extraction was separated, dried using MgSO₄, and then filtered. The filtrate was decompressed, and the resultant residue was washed using ether to obtain Compound PDQ A (0.53g, 87 %). The produced compound was identified by ¹H-NMR and MS.

¹H-NMR (500MHz, CD2Cl2) δ 8.10 (d, 4H), 7.85∼7.70 (m, 6H), 7.52 (d, 4H), 7.30∼7.15 (m, 3H), 4.5 (s, 2H).

MS (ESI -) m/z 151.0480, MS (ESI +) m/z 515.8274.

### Synthesis Example A: Synthesis of Base Resin 1 (HS/ECP)

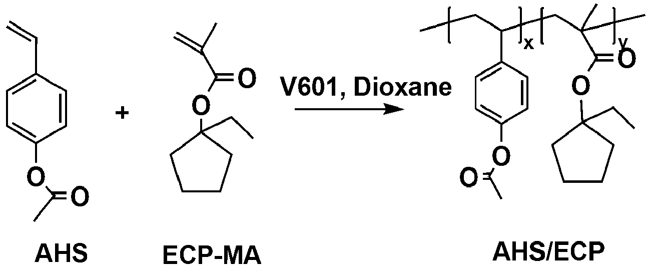

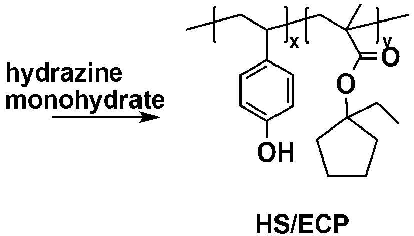

Acetoxystyrene (AHS) (3 g, 18.5 mmol), ethylcyclopentyl methacrylate (ECP-MA) (3.4 g, 18.5 mmol), and V601 (0.9 g, 3.7 mmol) were dissolved in 30 mL of dioxane, and a reaction was allowed to proceed at 80 °C for 4 hours. Here, hydrazine monohydrate (3 g) was added thereto, and the reaction was further allowed to proceed at room temperature for 2 hours. After completion of the reaction, 50 mL of distilled water and 5 g of acetic acid were added thereto, and an extraction process was performed thereon by using ethyl acetate. An organic layer thus obtained was collected and distilled under reduced pressure, and then allowed for precipitation by using n-hexane. The solid product thus obtained was dried at 40 °C for 24 hours to synthesize hydroxystyrene (HS)/ECP (x:y=5:5, Mw=5,000, and PDI=1.3). The results of ¹H-NMR analysis on the HS/ECP are shown in FIG. 5.

### Preparation Example 1: Preparation of Quencher Solution 1

The Base Resin 1 (HS/ECP) obtained in Synthesis Example A was dissolved in a PGME/PGMEA 7/3 (wt/wt) solution at 1.6 wt%, and 0.032 mmol of Compound PDQ A as a quencher was added thereto to prepare a low-molecular weight Quencher Solution 1.

### Comparative Preparation Example 1: Preparation of quencher-free solution

The Base Resin 1 (HS/ECP) obtained in Synthesis Example A was dissolved in a PGME/PGMEA 7/3 (wt/wt) solution at 1.6 wt% to prepare a quencher-free solution.

### Comparative Preparation Example 2: Preparation of comparative Quencher Solution 1

The Base Resin 1 (HS/ECP) obtained in Synthesis Example A was dissolved in a PGME/PGMEA 7/3 (wt/wt) solution at 1.6 wt%, and 0.032 mmol of Compound bi-iodized triphenyl sulfonium (BITPS)- Trifluoro(hydroxyethyl)methane sulfonamide (TSA)-Ad as a low-molecular weight quencher was added thereto to prepare comparative Quencher Solution 1. The BITPS-TSA-Ad thus obtained was confirmed by ¹H-NMR, and the results thereof are shown in FIG. 4.

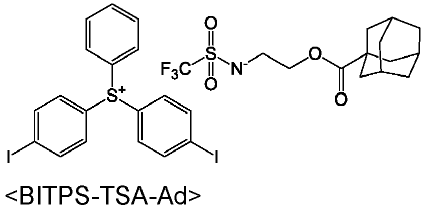

### Preparation Example A: Preparation of photoacid generator solution

The Base Resin 1 (HS/ECP) obtained in Synthesis Example A was dissolved in a PGME/PGMEA 7/3 (wt/wt) solution at 1.6 wt%, and 0.048 mmol of TPS/ perfluorobutanesulfonic acid (PFBS) as a photoacid generator was added thereto to prepare a photoacid generator solution.

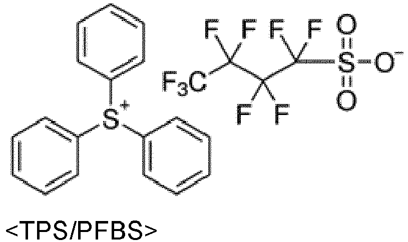

### Evaluation Example 1: Evaluation of acid diffusion length (ADL) and surface roughness (Rq)

### (ADL evaluation)

The ADL evaluation was performed by using the method disclosed in Macromolecules (43(9)4275 (published in 2010). In detail, the method was carried out as follows.

First, a 12-inch round silicon wafer board was pre-treated for 10 minutes under a UV Ozone (UVO) cleaning system. The silicon wafer board was spin-coated with the Quencher Solution 1 of Preparation Example 1 at a speed of 1,500 rpm for 30 seconds to form a first film having a thickness of 100 nm.

PDMS that was subjected to hydrophilization treatment by UVO cleaner equipment was spin-coated with the photoacid generator solution of Preparation Example A at a speed of 1,500 rpm for 30 seconds, and then, exposed to deep UV (DUV) rays having a wavelength of 248 nm at 250 mJ/cm² to form a second film. Due to the light exposure, an acid was generated by the photoacid generator in the second film.

Next, the second film overlapped with the first film so that the films were brought into contact with each other, and a pressure was applied thereto. Accordingly, the PDMS was removed while transferring the second film to the first film, thereby obtaining a laminate consisting of the silicon wafer board, the first film, and the second film. The laminate was maintained at 90 °C for 60 seconds to allow diffusion of the acid generated in the second film into the first film. Then, the laminate was washed with a tetramethyl ammonium hydroxide (TMAH) aqueous solution (2.38 wt%), and a thickness of the first film remained after the washing was measured to evaluate ADL.

The ADL for each sample was evaluated under the same conditions, except that, in forming the first film, each of the quencher-free solution of Comparative Preparation Example 1 and the comparative Quencher Solution 1 of Comparative Preparation Example 2 was used instead of the Quencher Solution 1 of Preparation Example 1, and the results thereof are shown in Table 1.

**Table 1**

| First film | Preparation Example 1 | Comparative Preparation Example 1 | Comparative Preparation Example 2 |
|---|---|---|---|
| ADL (nm) | 3.6 | 17 | 5.2 |

### (Rq evaluation)

The surface of the first film obtained immediately after spin-coating a silicon wafer board with a quencher solution was observed through an atomic force microscope, and Rq was calculated from the average value of the observed heights, and the Rq calculation results are expressed as Rq values before development in Table 2.

In addition, among the samples evaluated for the ADL, the surface of the first film newly exposed by the TMAH developing was observed through an atomic force microscope, and Rq was calculated from the average value of the observed heights, and the Rq calculation results are expressed as Rq values after development in Table 2.

**Table 2**

| First film | Preparation Example 1 | Comparative Preparation Example 1 | Comparative Preparation Example 2 |
|---|---|---|---|
| Rq (nm) before development | 0.267 | 0.234 | 0.241 |
| Rq (nm) after development | 0.508 | 1.910 | 0.515 |
| Change in Rq (nm) | 0.241 | 1.676 | 0.274 |

Referring to Tables 1 and 2, it was confirmed that the ADL value in Preparation Example 1 was significantly reduced compared to the ADL value in Comparative Preparation Example 2, and that the Rq value after development in Preparation Example 1 was similar with the Rq value after development in Comparative Preparation Example 2. In addition, the change in Rq value in Preparation Example 1 was small compared to the change in Rq value in Comparative Preparation Example 2, confirming that the change in surface roughness in Preparation Example 1 was small compared to the change in surface roughness in Comparative Preparation Example 2. Accordingly, it can be inferred that, when the acid generated by the light exposure was diffused, the quencher in Preparation Example 1 was diffused more evenly than in Comparative Preparation Example 2, so that the diffusion of the acid was prevented more evenly.

As described above, according to the one or more embodiments, a quencher having improved dispersibility and/or improved compatibility with a base resin and a photoresist composition including the quencher may be provided.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope as defined by the following claims.

## Claims

1. A carboxylate salt represented by Formula 1: 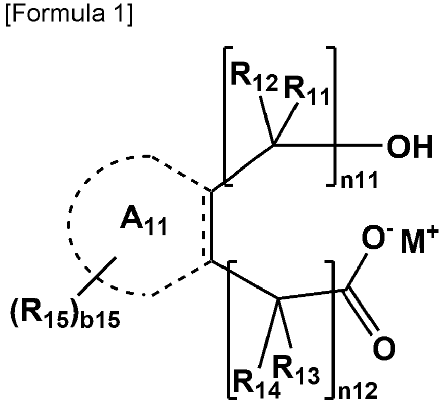 wherein, in Formula 1,
A₁₁ is a cyclic C₁-C₂₀ hydrocarbon group that selectively includes a heteroatom, and 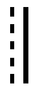 represents at least one of a carbon-carbon single linkage or a carbon-carbon double linkage,
R₁₁ to R₁₅ are each independently at least one of hydrogen; deuterium; a halogen; or a linear, branched, or cyclic C₁-C₂₀ monovalent hydrocarbon group that optionally includes a heteroatom,
b15 is an integer from 0 to 10,
n11 and n12 are each 0, 1, or 2 such that the sum of n11 and n12 is 1 or 2, and
M⁺ is a at least one of a substituted or unsubstituted sulfonium cation, a substituted or unsubstituted iodonium cation, or a substituted or unsubstituted ammonium cation.

2. The carboxylate salt of claim 1, wherein A₁₁ a cyclic C₁-C₁₀ hydrocarbon group.

3. The carboxylate salt of claims 1 or 2, wherein A₁₁ is at least one of a cyclopentane group, a cyclopentene group, a cyclopentadiene group, a cyclohexane group, a cyclohexene group, a cyclohexadiene group, a benzene group, or a naphthalene group.

4. The carboxylate salt of any of claims 1-3, wherein A₁₁ is at least one of a cyclopentane group, a cyclohexane group, a benzene group, or a naphthalene group.

5. The carboxylate salt of any of claims 1-4, wherein the carboxylate salt is represented by at least one of Formulae 1-1 to 1-5: 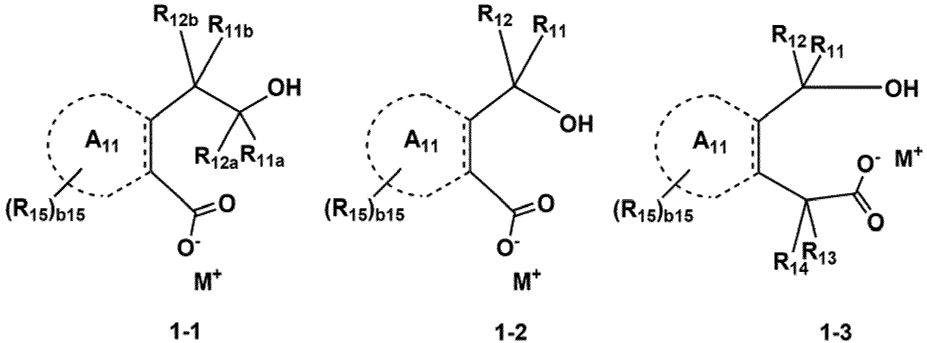 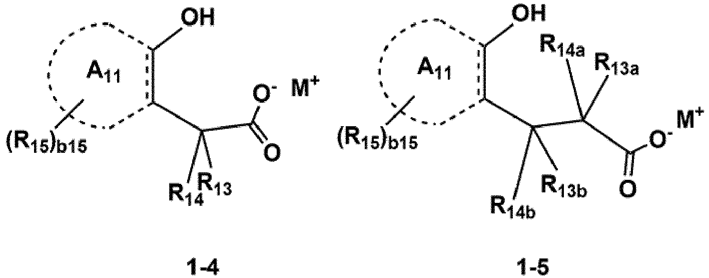 wherein, in Formulae 1-1 to 1-5,
R₁₁ₐ, R₁₂ₐ, R₁₃ₐ, R₁₄ₐ, R_{11b}, R_{12b}, R_{13b}, and R_{14b} are each at least one of hydrogen; deuterium; a halogen; or a linear, branched, or cyclic C₁-C₂₀ monovalent hydrocarbon group that optionally includes a heteroatom;
preferably wherein A₁₁ is at least one of a cyclopentane group, a cyclohexane group, a benzene group, or a naphthalene group.

6. The carboxylate salt of any of claims 1-5, wherein M⁺ is represented by at least one of Formulae 3-1 to 3-3: 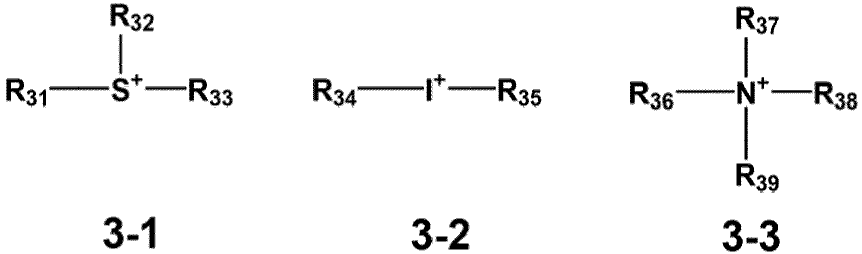 wherein, in Formulae 3-1 to 3-3,
R₃₁ to R₃₉ are each independently at least one of a substituted or unsubstituted C₁-C₂₀ alkyl group or a substituted or unsubstituted C₃-C₂₀ carbocyclic group,
two adjacent groups among R₃₁ to R₃₃ are optionally bonded to each other to form a ring,
R₃₄ and R₃₅ are optionally bonded to each other to form a ring, and
two adjacent groups among R₃₆ to R₃₉ are optionally bonded to each other to form a ring;
preferably wherein
R₃₁ to R₃₅ are each independently a C₆-C₂₀ aryl group substituted with at least one of deuterium, a halogen, a hydroxy group, a C₁-C₆ alkyl group, aC₁-C₆ halogenated alkyl group, a C₁-C₆ alkoxy group, a C₃-C₆ cycloalkyl group, or a C₃-C₆ cycloalkoxy group, and
R₃₆ to R₃₉ are each independently a C₁-C₁₀ alkyl group unsubstituted or substituted with at least one of deuterium, a halogen, a hydroxyl group, a C₁-C₆ alkyl group, a C₁-C₆ alkoxy group, a C₃-C₆ cycloalkyl group, a C₃-C₆ cycloalkoxy group, or a C₆-C₁₀ aryl group,
two adjacent groups among R₃₁ to R₃₃ are optionally bonded to each other to form a ring,
R₃₄ and R₃₅ are optionally bonded to each other to form a ring, and
two adjacent groups among R₃₆ to R₃₉ are optionally bonded to each other to form a ring.

7. The carboxylate salt of any of claims 1-6, wherein M⁺ is represented by at least one of Formulae 3-11 to 3-13: 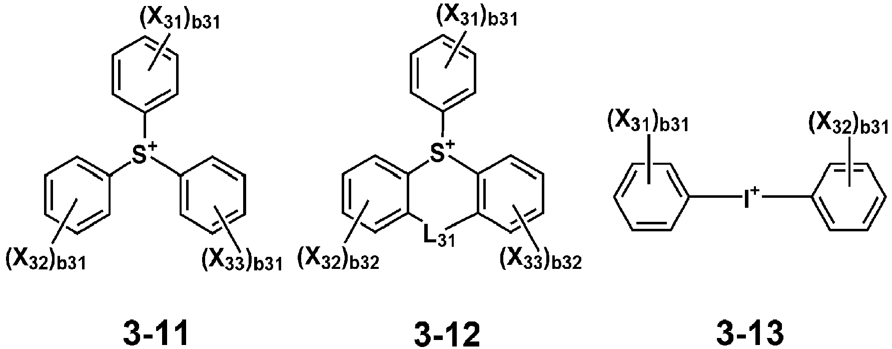 wherein, in Formulae 3-11 to 3-13,
X₃₁ to X₃₃ are each independently, at least one of hydrogen, deuterium, a halogen, a C₁-C₆ alkyl group, or a C₁-C₆ halogenated alkyl group,
b31 is an integer from 1 to 5,
b32 is an integer from 1 to 4,
L₃₁ is at least one of a single bond, O, S, CO, SO, SO₂, CRR', or NR, and
R and R' are each independently, at least one of hydrogen, deuterium, a halogen, a hydroxy group, a C₁-C₆ alkyl group, a C₁-C₆ halogenated alkyl group, a C₁-C₆ alkoxy group, a C₃-C₆ cycloalkyl group, or a C₃-C₆ cycloalkoxy group.

8. The carboxylate salt of any of claims 1-7, wherein the carboxylate salt is represented by at least one of Formulae 1-11 to 1-20: 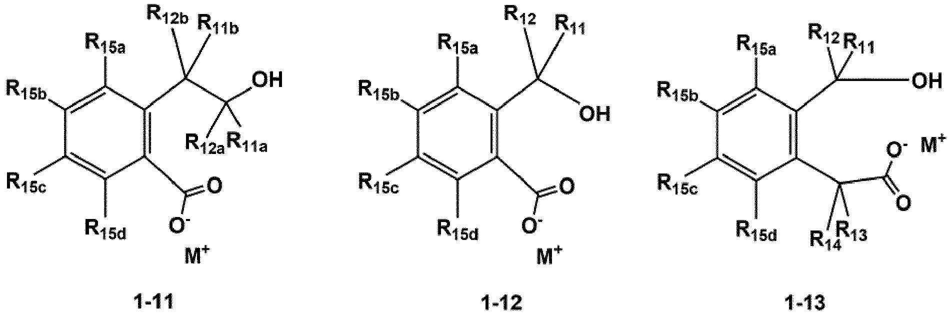 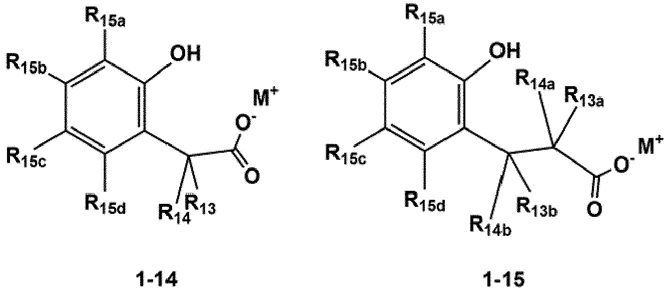 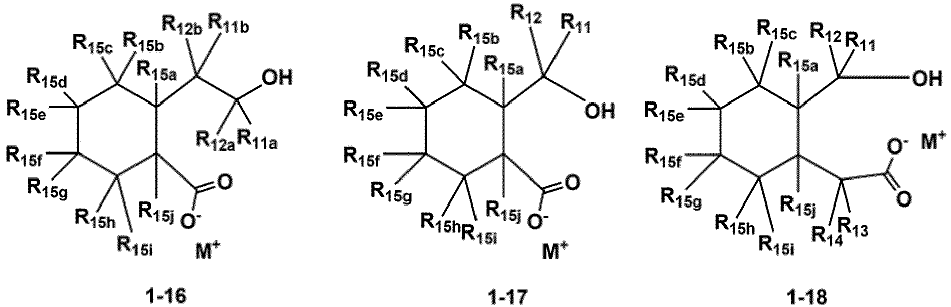 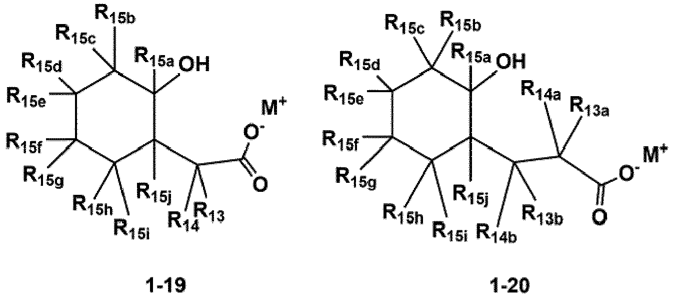 wherein, in Formulae 1-11 to 1-20,
R₁₁ₐ, R₁₂ₐ, R₁₃ₐ, R₁₄ₐ, R_{11b}, R_{12b}, R_{13b}, R_{14b}, and R₁₅ₐ to R₁₅ⱼ are each at least one of hydrogen; deuterium; a halogen; or a linear, branched, or cyclic C₁-C₂₀ monovalent hydrocarbon group that optionally includes a heteroatom;
preferably wherein M+ is represented by one of Formulae 3-11 to 3-13: 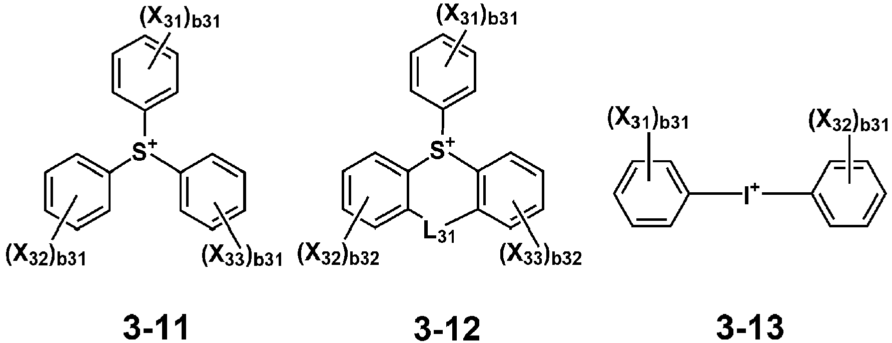 wherein, in Formulae 3-11 to 3-13,
X₃₁ to X₃₃ are each independently, at least one of hydrogen, deuterium, a halogen, a C₁-C₆ alkyl group, or a C₁-C₆ halogenated alkyl group,
b31 is an integer from 1 to 5,
b32 is an integer from 1 to 4,
L₃₁ is at least one of a single bond, O, S, CO, SO, SO₂, CRR', or NR, and
R and R' are each independently, at least one of hydrogen, deuterium, a halogen, a hydroxy group, a C₁-C₆ alkyl group, a C₁-C₆ halogenated alkyl group, a C₁-C₆ alkoxy group, a C₃-C₆ cycloalkyl group, or a C₃-C₆ cycloalkoxy group.

9. A photoresist composition comprising the carboxylate salt of any of claims 1-8, an organic solvent, a base resin, and a photoacid generator.

10. The photoresist composition of claim 9, wherein the carboxylate salt is configured to be a photodegradable compound that generates an acid during light exposure and acts as a quenching base for neutralizing an acid before the light exposure; and/or
wherein an amount of the carboxylate salt is in a range of 0.1 parts by weight to 40 parts by weight based on 100 parts by weight of the base resin.

11. The photoresist composition of claims 9 or 10, wherein the base resin includes a repeating unit represented by Formula 4: 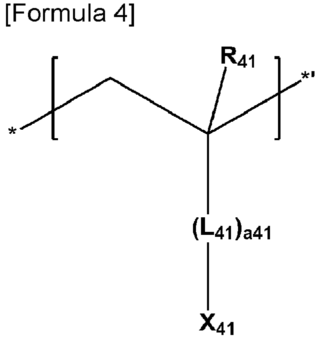 wherein, in Formula 4,
R₄₁ is at least one of a hydrogen, deuterium, a halogen, a linear or branched C₁-C₆ alkyl group, or a linear or branched C₁-C₆ halogenated alkyl group,
L₄₁ is at least one of a single bond, a substituted or unsubstituted C₁-C₁₀ alkylene group, a substituted or unsubstituted C₃-C₁₀ cycloalkylene group, a substituted or unsubstituted C₃-C₁₀ heterocycloalkylene group, a substituted or unsubstituted phenylene group, a substituted or unsubstituted naphthylene group, *-O-*', *-C(=O)O-*', -OC(=O)-*', *-C(=O)NH-*', -NHC(=O)-*', or a combination thereof,
a41 is an integer from 1 to 6,
X₄₁ is an acid labile group, and
* and *' each indicate a binding site to a neighboring atom;
preferably wherein the base resin further includes a second repeating unit represented by Formula 5: 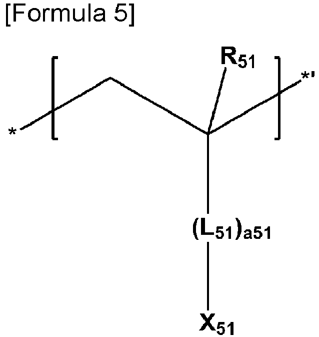
wherein, in Formula 5,
R₅₁ is at least one of hydrogen, deuterium, a halogen, a linear or branched C₁-C₆ alkyl group, or a linear or branched C₁-C₆ halogenated alkyl group,
L₅₁ is at least one of a single bond, a substituted or unsubstituted C₁-C₁₀ alkylene group, a substituted or unsubstituted C₃-C₁₀ cycloalkylene group, a substituted or unsubstituted C₃-C₁₀ heterocycloalkylene group, a substituted or unsubstituted phenylene group, a substituted or unsubstituted naphthylene group, *-O-*', *-C(=O)O-*', -OC(=O)-*', *-C(=O)NH-*', -NHC(=O)-*', or a combination thereof,
a51 is an integer from 1 to 6, and
X₅₁ is a non-acid labile group.

12. The photoresist composition of any of claims 9-11, wherein the photoacid generator includes a sulfonium salt, an iodonium salt, or a combination thereof.

13. The photoresist composition of any of claims 9-12, wherein the photoacid generator is represented by Formula 7:
[Formula 7] B₇₁⁺ A₇₁⁻
wherein, in Formula 7,
B₇₁⁺ is represented by Formula 7A, and A₇₁⁻ is represented by at least one of Formulae 7B to 7D, and
B₇₁⁺ and A₇₁⁻ are linked via an ionic bond or a carbon-carbon covalent bond: 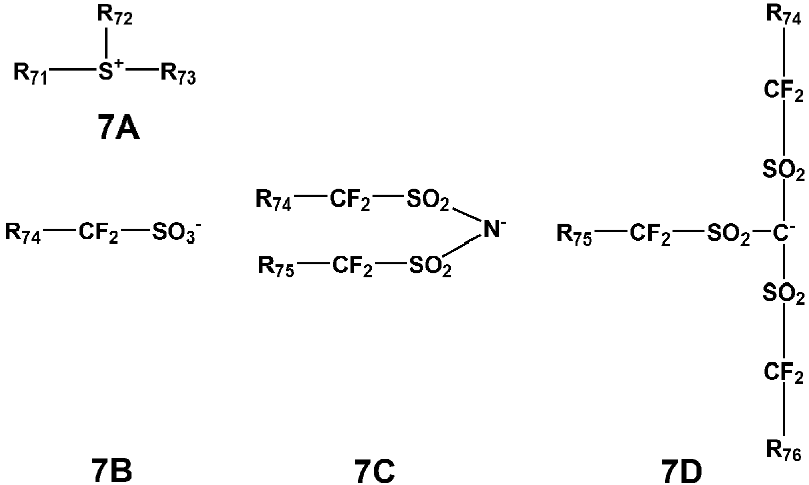 wherein, in Formulae 7A to 7D,
R₇₁ to R₇₃ are each independently at least one of a linear, branched, or cyclic C₁-C₂₀ monovalent hydrocarbon group,
two adjacent groups of R₇₁ to R₇₃ are optionally bonded to each other to form a ring, and
R₇₄ to R₇₆ are each independently at least one of fluorine (F), or a linear, branched, or cyclic C₁-C₂₀ monovalent hydrocarbon group that optionally includes a heteroatom.

14. A method of forming a pattern, the method comprising:
forming a photoresist film by coating a board with the photoresist composition of any of claims 9-13;
exposing at least a portion of the photoresist film with high energy radiation; and
developing the exposed photoresist film using a developing solution.

15. The method of claim 14, wherein the exposing is performed by irradiating the portion of the photoresist film with at least one of a krypton fluoride (KrF) excimer laser, an argon fluoride (ArF) excimer laser, an extreme ultraviolet (EUV), or an electron beam (EB).
